# EUROPEAN PATENT APPLICATION

(11) **EP 4 122 444 A1**
(43) Date of publication of application: **25.01.2023**
(21) Application number: 21770632.4
(22) Date of filing: 22.03.2021
(51) Int. Cl.: A61K 8/98, A61K 35/16, A61P 19/02

(54) **COMPOSITION CONTAINING MITOCHONDRIA AND USE THEREOF FOR REPAIRING CARTILAGE DAMAGE OR AMELIORATING DEGENERATIVE ARTHRITIS**

(30) Priority: 20.03.2020 US 202062992546 P
(71) Applicant: Taiwan Mitochondrion Applied Technology Co. Ltd., Zhubei City, Hsinchu County, Taiwan 302058 (TW)
(72) Inventor: CHENG, Han-Chung, Zhubei City Hsinchu County, Taiwan 302 (TW); HSU, Chih-Kai, Zhubei City Hsinchu County, Taiwan 302 (TW); YANG, Shun-Chieh, Zhubei City Hsinchu County, Taiwan 302 (TW); CHENG, An-Ling, Zhubei City Hsinchu County, Taiwan 302 (TW); TU, Chi-Tang, Zhubei City Hsinchu County, Taiwan 302 (TW)
(74) Representative: dompatent von Kreisler Selting Werner - Partnerschaft von Patent- und Rechtsanwälten mbB
(86) International application number: PCT/CN2021/082094
(87) International publication number: WO 2021/185376

(57) **Abstract**

The embodiments of the present disclosure provide a composition including mitochondria. By providing the mitochondria to cartilage, the composition can improve the mitochondrial function of the chondrocytes in the cartilage so as to increase the repair ability of the chondrocytes and repair the cartilage damage caused by aging of the chondrocytes, thereby achieving the purpose of treating osteoarthritis.

## Description

### 1. Technical Field

The present disclosure relates to a use of mitochondria and a composition including mitochondria, specifically, to a use of mitochondria in manufacturing a composition for repairing cartilage damage or relieving the symptoms of osteoarthritis and a composition including mitochondria and platelet-rich plasma.

### 2. Related Art

Osteoarthritis, also known as degenerative joint disease, is the most common joint disease in human aging. The main lesions of osteoarthritis occur in the interface of joints between bones, that is the so-called articular cartilage. In the X-ray images of the patient with osteoarthritis, cartilage damage, uneven joint surface, narrow articular cavity, and formation of bone spur can be seen. The main symptoms of osteoarthritis include pain, rigidity, swelling, inflammation, or deformation. The general osteoarthritis treatment includes drug therapy, physical therapy, hyaluronic acid injection, artificial joint replacement, and so on.

However, except for artificial joint replacement surgery, other therapies can only temporarily relieve inflammation and pain but cannot effectively slow or delay the disease progression. Therefore, how to relieve and treat osteoarthritis is one goal of the research.

### SUMMARY

In one embodiment of the present disclosure, a use of mitochondria in manufacturing a composition for repairing cartilage damage is provided.

In one embodiment of the present disclosure, a use of mitochondria in manufacturing a composition for relieving osteoarthritis is provided.

In one embodiment of the present disclosure, a composition comprising mitochondria and growth factors from blood is provided.

### BRIEF DESCRIPTION OF THE DRAWINGS

The present disclosure will become more fully understood from the detailed description given hereinbelow and the accompanying drawings which are given by way of illustration only and thus are not limitative of the present disclosure and wherein:
FIG. 1 is a chart of the cell viability of the chondrosarcoma cells damaged with 75 µM tBHP and repaired by the composition of the embodiments of the present disclosure;
FIG. 2 is a chart of the senescence level of the chondrosarcoma cells damaged with 50 µM tBHP and repaired by the composition of the embodiments of the present disclosure;
FIG. 3 is a staining image of the chondrosarcoma cells damaged with 50 µM tBHP and repaired by the composition of the embodiments of the present disclosure; and
FIG. 4 is mitochondrial membrane potential analysis diagrams of the chondrosarcoma cells damaged with 50 µM tBHP and repaired by the composition of the embodiments of the present disclosure.

### DETAILED DESCRIPTION

In the following detailed description, for purposes of explanation, numerous specific details are set forth in order to provide a thorough understanding of the disclosed embodiments. According to the description, claims and the drawings disclosed in the specification, one skilled in the art may easily understand the concepts and features of the present disclosure. The following embodiments further illustrate various aspects of the present disclosure, but are not meant to limit the scope of the present disclosure.

Mitochondria are places where oxidative phosphorylation (OXPHOS) and adenosine triphosphate (ATP) synthesis occur. In addition to supplying the energy for normal cell metabolism, the mitochondria are also responsible for regulating the functions including dealing with the oxidative stress in cells, signaling, and so on. Compared to the normal chondrocytes, the mitochondrial function of the damaged or aged chondrocytes is significantly reduced, specifically, ATP production efficiency is decreased, the membrane potential is decreased, the efficiency of the electron transport chain is decreased, and so on. With decreasing in the mitochondrial function, various bio reactions become declined, that is, the chondrocytes begin to age and are gradually unable to maintain normal function, and then the symptoms of osteoarthritis and inflammation appear. Therefore, by providing healthy mitochondria to the chondrocytes, the senescence level of the chondrocytes can be reduced and the repair ability of the chondrocytes can be increased, thereby to treat the diseases such as osteoarthritis caused by aging.

One embodiment of the present disclosure provides a composition for repairing cartilage damage or relieving osteoarthritis which includes mitochondria. In this embodiment, the concentration of the mitochondria may be 1 µg/mL to 4000 µg/mL, but it is not limited thereto. In other embodiment, the concentration of the mitochondria may be 1 µg/mL to 200 µg/mL. In another embodiment, the concentration of the mitochondria may be 1 µg/mL to 100 µg/mL. In still another embodiment, the concentration of the mitochondria may be 1 µg/mL to 40 µg/mL. In further another embodiment, the concentration of the mitochondria may be 15 µg/mL to 40 µg/mL. In this embodiment, the effective dose of the mitochondria may be 7.9 mg to 2.11 g per 1 cm² of the inner surface area of the articular cavity, but it is not limited thereto. In other embodiment, the effective dose of the mitochondria may be 7.9 mg to 21.1 mg per 1 cm² of the inner surface area of the articular cavity.

The mitochondria included in the composition may be exogenous mitochondria. The exogenous mitochondria are taken from the cells other than the one to which the composition is applied. The one to which the composition is applied and the exogenous mitochondria provider are preferably the same genus, and more preferably the same species. In other embodiment, the mitochondria may be autologous mitochondria taken from the one to which the composition is applied to. After obtaining the cells for providing the mitochondria, the mitochondria may be isolated directly from the cells, or the mitochondria may be isolated from the cells after culturing the cells in vitro. The cells for providing the exogenous mitochondria or the autologous mitochondria may be the cells having the mitochondria, such as adipose stem cells, monocytes, embryonic stem cells, mesenchymal stem cells, hematopoietic stem cells, CD34+ stem cells, bone marrow stem cells, and so on.

The composition of the present disclosure may be administrated to joints by oral route or injection. When the composition is administrated to joints by injection, the composition is injected into articular cavity, and the composition contacts the chondrocytes in the articular cavity and enters the chondrocytes.

In other embodiment, the composition further includes growth factors from blood. The growth factors may be derived from platelet-rich plasma (PRP). The growth factor may include TGF-β1, PDGF-AA, PDGF-AB, or PDGF-BB. The platelet-rich plasma as described here includes 1×10⁹ of platelets per milliliter and various growth factors, wherein the concentration of TGF-β1 may be 7767 pg/mL or more, the concentration of PDGF-AA may be 9.73 pg/mL or more, the concentration of PDGF-AB may be 10 pg/mL or more, and the concentration of PDGF-BB may be 590 pg/mL or more. In the composition, there may be 1 µg to 40 µg of the mitochondria per 10 µL of PRP, that is, the ratio of the mitochondria to PRP may be 1 µg: 10 µL to 40 µg:10 µL, but it is not limited thereto. In other embodiment, there may be 15 µg to 40 µg of the mitochondria per 10 µL of PRP in the composition, that is, the ratio of the mitochondria to PRP may be 15 µg:10 µL to 40 µg:10 µL. In the composition, the concentration of the mitochondria may be 1000 µg/mL to 4000 µg/mL, but it is not limited thereto. In other embodiment, the concentration of the mitochondria may be 1500 µg/mL to 4000 µg/mL. In this embodiment, the effective dose of the mitochondria may be 7.9 mg to 2.11 g per 1 cm² of the inner surface area of the articular cavity, but it is not limited thereto. In other embodiment, the effective dose of the mitochondria may be 7.9 mg to 21.1 mg per 1 cm² of the inner surface area of the articular cavity.

In other embodiment, the composition may further include a pharmaceutically acceptable carrier. The pharmaceutically acceptable carrier includes any carrier used in standard medical products or cosmetic products, and the carrier may be liquid or semisolid depending on the form of the composition. For example, the carrier may include but is not limited to hyaluronic acid, gelatin, emulsifier, water, normal saline, buffered saline, or ethanol, which does not affect the function of the composition of the present disclosure.

The following describes how to prepare the composition of the present disclosure.

### Extraction of mitochondria

The mitochondria used in one embodiment of the present disclosure are taken from human adipose-derived stem cells (ADSC). The culture medium for the stem cells includes Keratinocyte SFM (1X) solution (Gibco), bovine pituitary extract (BPE, Gibco), and 10% (%w/w) FBS (HyClone). Firstly, ADSCs are cultured in a Petri dish to 1.5×10⁸ cells and then washed with Dulbecco's phosphate-buffered saline (DPBS). Next, DPBS is removed, trypsin for dissociating adherent cells from the Petri dish surfaces is added and reacted at 37°C for 3 min, and the reaction is stopped by adding the culture medium for the stem cells. Next, ADSCs are washed down from the Petri dish, dispersed, and centrifuged at 600 g for 10 min, and the supernatant is removed. Next, the remained ADSCs and 80 mL of IBC-1 buffer (225 mM mannitol, 75 mM sucrose, 0.1 mM EDTA, and 30 mM Tris-HCl pH 7.4) are added to a homogenizer, and ADSCs are ground 15 times on ice by the homogenizer. Next, the ground ADSCs are centrifuged at 1000 g for 15 min, and the supernatant is collected in another centrifuge tube and centrifuged again at 9000 g for 10 min. After centrifuging, the supernatant is removed, and the obtained pellets are the mitochondria. Next, 1.5 mL of IBC-2 buffer (225 mM mannitol, 75 mM sucrose, and 30 mM Tris-HCl pH 7.4) and a protease inhibitor are added to the mitochondria, and then the mitochondria are stored at 4°C.

### Experiment 1: repairing chondrocyte damage

In this experiment, the chondrosarcoma cells (SW-1535) are induced to be damaged by 75 µM of tBHP. The effect of the composition including the mitochondria on repairing the damage of SW-1535 cells is assessed by Alamar blue cell viability reagent kit and expressed by the cell viability (%).

The chondrosarcoma cells (SW-1535) are often used for studying the mechanism of cartilage damage and are a model for the development of chondrocytes. The SW1535 cells are cultures in the culture medium for the chondrosarcoma cells at the circumstance with 0% CO₂. The culture medium for the chondrosarcoma cells includes 90 %v/v Leibovitz's L-15, 2mM L-glutamine, and 10 %v/v fetal bovine serum.

tBHP (tert-butyl hydroperoxide) is an organic hydroperoxide and is often used as a substance for inducing cellular oxidative stress damage, aging, and apoptosis. tBHP is used as a substance for inducing SW-1535 cells to be aging or damaged in this experiment.

Alamar blue is a reagent for assessing cell viability. Resazurin in the Alamar blue reagent is a redox indicator and is a deep blue dye that is non-toxic, permeable to the cell membrane, and low-fluorescent. When resazurin enters into the healthy cells, it is reduced to resorufin, which is pink and high-fluorescent, due to the reduced environment in the cells. The cell viability may be assessed by measuring the absorbance or the fluorescence of resorufin. The higher the absorbance or the fluorescence of resorufin indicates the higher cell viability. The higher cell viability indicates the healthier cells and the better proliferation ability. The better proliferation ability indicates the more cells. Therefore, Alamar blue is used as an indicator for assessing cell proliferation or cell viability in this experiment.

The following describes the experimental procedure. Firstly, the chondrosarcoma cells (SW-1535) passage numbers at 4 to 10 are used for experiments. When SW-1535 cells are cultured to 80% full of the Petri dish, the cell culture medium is removed, and then the cells are rinsed with phosphate buffered saline (PBS). Next, 0.25% trypsin is added to the Petri dish and reacted at 37°C for 5 min, and the reaction is stopped by adding the culture medium into the Petri dish. Next, the SW-1535 cells and the culture medium in the Petri dish are moved to a centrifuge tube and centrifuged at 1000 rpm (300 g) for 5 min, and then the supernatant is removed. Next, the fresh culture medium is added into the centrifuge tube, and then the cells are counted. Next, SW-1535 cells are cultured at a density of 2×10⁴ cells per well with the culture medium in a 24-well plate for 16 hours. Next, tBHP is added to the 24-well plate, and SW-1535 cells are cultured in the culture medium including 75 µM tBHP for 4 hours. Next, the culture medium including 75 µM tBHP is removed, and the culture medium including the composition with different concentrations of the mitochondria is added, and SW-1535 cells are cultured in the culture medium including the composition with different concentrations of the mitochondria for 20 hours. The concentrations of the mitochondria in the well are 1 µg/mL, 15 µg/mL, or 40 µg/mL. After cell culture, SW-1535 cells are washed with PBS, and the culture medium is replaced with the medium including Alamar blue and further cultured for 3 hours. After cell culturing with the medium including Alamar blue, the cell viability for SW-1535 cells is calculated by the fluorescence measured at OD530/595.

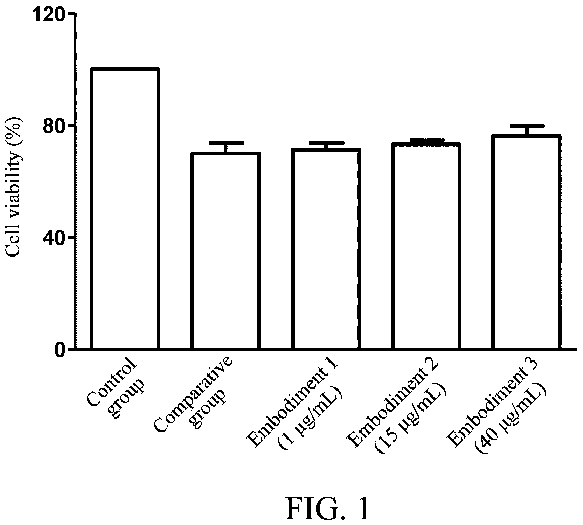

The experiment results of the cell viability of SW-1535 cells are shown in Table 1 and FIG. 1. FIG. 1 is a chart of the cell viability of the chondrosarcoma cells damaged with 75 µM tBHP and repaired by the composition of the embodiments of the present disclosure. The control group is the group in which SW-1535 cells are not induced to be damaged by tBHI'. The comparative group is the group in which SW-1535 cells are induced to be damaged by tBHP and are not treated with the composition of the embodiments of the present disclosure. The test group is the group in which SW-1535 cells are induced to be damaged by tBHP and are treated with the compositions of the embodiments including 1 µg/mL, 15 µg/mL, or 40 µg/mL of the mitochondria. The vertical axis is the cell number ratio compared to the control group and expressed by the cell viability (%). The experiment results show that the cell viability of the test group is higher than that of the comparative group, and the cell viability increases with the concentration of the mitochondria increased. This indicates the composition including the mitochondria indeed reduces the damage and death of SW-1535 cells caused by tBHI'.

**Table 1**

| Group | | Mitochondria (µg/mL) | tBHP (µM) | Cell viability (%) |
|---|---|---|---|---|
| Control group | | - | - | 100 |
| Comparative group | | - | 75 | 69.9±5.5 |
| Test group | Embodiment 1 | 1 | | 71.2±4.2 |
| | Embodiment 2 | 15 | | 73.1±3.1 |
| | Embodiment 3 | 40 | | 76.3±6.1 |

### Experiment 2: reducing the senescence level of the chondrocytes

In this experiment, the SW-1535 cells are induced to be aged by tBHP. The effect of the composition including the mitochondria on reducing the senescence level of the chondrocytes caused by tBHP is assessed by SA-β-gal kit and expressed by the senescence level (%).

In the aged cells, senescence-associated beta-galactosidase (SA-β-gal) is overexpressed, and thus SA-β-gal may be a biomarker of cellular senescence level. The senescence level of SW-1535 cells is assessed by a SA-β-gal kit (Senescence β-Galactosidase Staining Kit #9860, Cell Signaling Technology) in this experiment.

The experimental procedure in this experiment is generally the same as the experimental procedure in Experiment 1. The difference is that SW-1535 cells are cultured at a density of 4×10⁴ cells per well with the culture medium in a 12-well plate for 16 hours. Next, tBHP is added to the 12-well plate, and SW-1535 cells are cultured in the culture medium including 50 µM tBHP for 4 hours. Next, the culture medium including tBHP is removed, and the culture medium including the composition with different concentrations of the mitochondria is added, and SW-1535 cells are cultured in the culture medium including the composition with different concentrations of the mitochondria for 20 hours. The concentrations of the mitochondria in the well are 1 µg/mL, 15 µg/mL, or 40 µg/mL. After cell culture, SW-1535 cells are washed with PBS, and the senescence level is assessed by SA-β-gal kit.

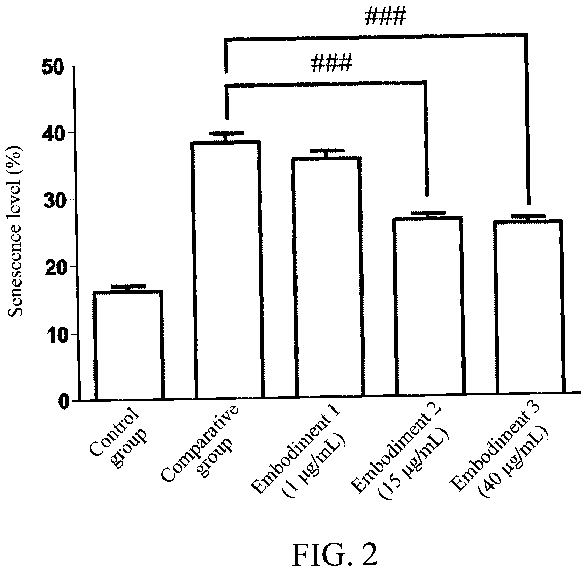

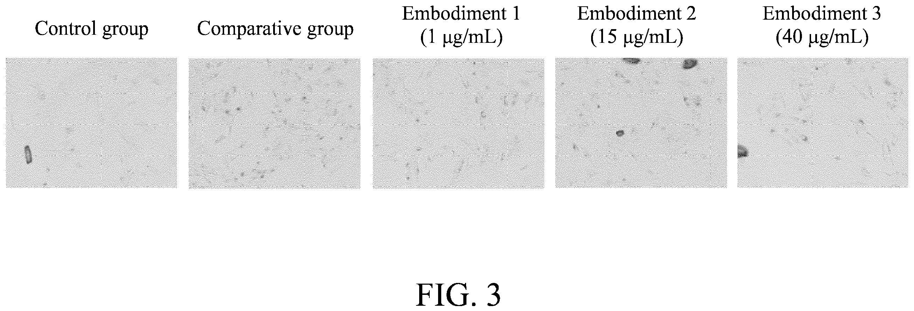

The senescence level of SW-1535 cells, as the experiment results, are shown in Table 2 and FIGs. 2 and 3. FIG. 2 is a chart of the senescence level of the chondrosarcoma cells damaged with 50 µM tBHP and repaired by the composition of the embodiments of the present disclosure. FIG. 3 is a staining image of the chondrosarcoma cells damaged with 50 µM tBHP and repaired by the composition of the embodiments of the present disclosure. The control group is the group in which SW-1535 cells are not induced to be damaged by tBHI'. The comparative group is the group in which SW-1535 cells are induced to be damaged by tBHP and are not treated with the composition of the embodiments of the present disclosure. The test group is the group in which SW-1535 cells are induced to be damaged by tBHP and are treated with the compositions of the embodiments including 1 µg/mL, 15 µg/mL, or 40 µg/mL of the mitochondria. The vertical axis is the senescence level (%), and the senescence level indicates the percentage of the amount of the aged cells to the amount of all cells. ### indicates a statistically significant difference (P<0.01) compared to the comparative group. The experiment results show that the senescence level of the test group is lower than that of the comparative group, and the senescence level decreases with the concentration of the mitochondria increased. This indicates the composition including the mitochondria indeed reduces the senescence level of SW-1535 cells caused by tBHI'.

**Table 2**

| Group | | Mitochondria (µg/mL) | tBHP (µM) | Senescence level (%) |
|---|---|---|---|---|
| Control group | | - | - | 16.2±2.3 |
| Comparative group | | - | 50 | 38.2±3.8 |
| Test group | Embodiment 1 | 1 | | 35.5±2.6 |
| | Embodiment 2 | 15 | | 26.3±2.5 |
| | Embodiment 3 | 40 | | 25.6±2.4 |

### Experiment 3: improving the mitochondrial function of the chondrocytes

In this experiment, the SW-1535 cells are induced to be aged by 50 µM of tBHI'. The mitochondrial function of the chondrocytes is assessed by the mitochondrial membrane potential.

The mitochondrial membrane potential analysis is to determine the mitochondrial function by measuring the change of the mitochondrial membrane potential. Tetramethylrhodamine ethyl ester (TMRE) is a positively charged fluorescent dye, and it may accumulate in the active mitochondria so that TMRE may be used for marking healthy mitochondria. When the mitochondria are not active or depolarized, the mitochondrial membrane potential decreases, and TMRE cannot retain in the mitochondria. Carbonylcyanide 4-(trifluoromethoxy)phenylhydrazone (FCCP) is an ionophore permeable to the mitochondrial inner membrane, and it combines proton and disrupts ATP synthesis to lead to a change in membrane potential. FCCP may be used for dissipating mitochondrial membrane potential so that FCCP is often used for the comparative group in which the mitochondria are deactivated or depolarized. The change in the mitochondrial membrane potential may be obtained by staining by TMRE and FCCP and analyzing the fluorescence intensity, which may serve as the basis for determining the mitochondrial function. The supernatant after treating with TMRE may be detected by flow cytometry, and the proportion of the functional mitochondria to all particles in the supernatant may be analyzed according to TMRE marking.

The experimental procedure in this experiment is generally the same as the experimental procedure in Experiment 1. The difference is that SW-1535 cells are cultured at a density of 1×10⁵ cells per well with the culture medium in a 6-well plate for 16 hours. Next, tBHP is added to the 6-well plate, and SW-1535 cells are cultured in the culture medium including 50 µM tBHP for 4 hours. Next, the culture medium including tBHP is removed, and the culture medium including the composition with different concentrations of the mitochondria is added, and SW-1535 cells are cultured in the culture medium including the composition with different concentrations of the mitochondria for 20 hours. The concentrations of the mitochondria in the well are 1 µg/mL, 15 µg/mL, or 40 µg/mL. After cell culture, SW-1535 cells are washed with PBS, and then SW-1535 cells are stained by TMRE and FCCP and analyzed by flow cytometry.

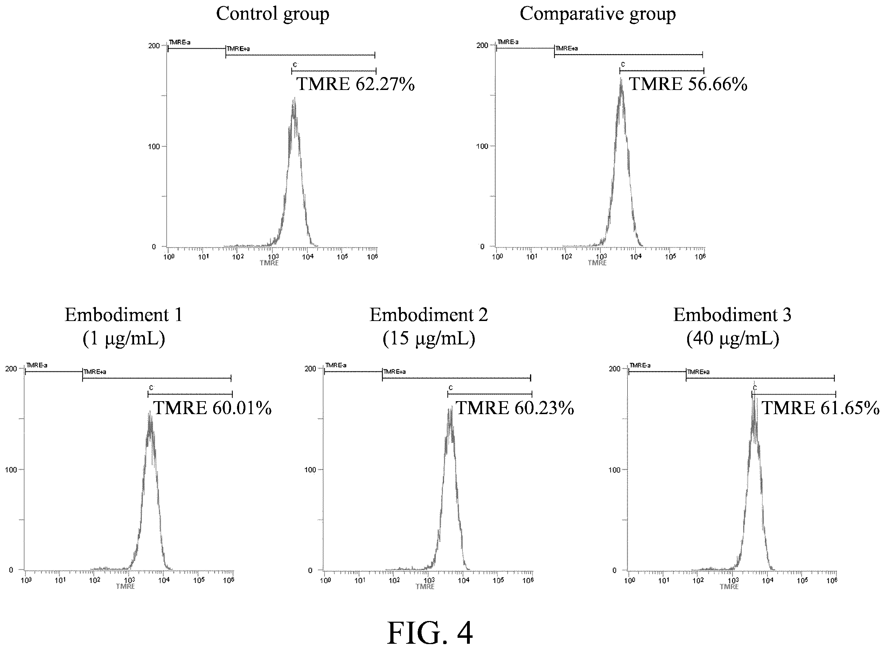

The experiment results of the mitochondrial membrane potential analysis of SW-1535 cells are shown in Table 3 and FIG. 4. FIG. 4 is mitochondrial membrane potential analysis diagrams of the chondrosarcoma cells damaged with 50 µM tBHP and repaired by the composition of the embodiments of the present disclosure. The control group is the group in which SW-1535 cells are not induced to be damaged by tBHI'. The comparative group is the group in which SW-1535 cells are induced to be damaged by tBHP and are not treated with the composition of the embodiments of the present disclosure. The test group is the group in which SW-1535 cells are induced to be damaged by tBHP and are treated with the compositions of the embodiments including 1 µg/mL, 15 µg/mL, or 40 µg/mL of the mitochondria. The experiment results show that the amount of the functional mitochondria of the test group is more than that of the comparative group, and the membrane potential of the test group returns to close to the control group with the concentration of the mitochondria increased. This indicates the composition including the mitochondria indeed improves the mitochondrial function of SW-1535 cells, and the improvement of the mitochondrial function further indicates the cells have excellent anti-aging and repairing ability.

**Table 3**

| Group | | Mitochondria (µg/mL) | tBHP (µM) | Membrane potential analysis (%) |
|---|---|---|---|---|
| Control group | | - | - | 62.27 |
| Comparative group | | - | 50 | 56.66 |
| Test group | Embodiment 1 | 1 | | 60.01 |
| | Embodiment 2 | 15 | | 60.23 |
| | Embodiment 3 | 40 | | 61.65 |

### Experiment 4: relieving osteoarthritis

In this experiment, the mice are induced to suffer from osteoarthritis by monosodium iodoacetate (MIA). The effect of the composition including the mitochondria and the composition including the mitochondria and PRP on the recovery of the motor coordination of the mice with osteoarthritis is assessed by the rotarod test. C57BL/6 male mice (age: 8 week-old; weight: 18 to 22 g) is used in this experiment.

Monosodium iodoacetate (MIA), an inhibitor of glyceraldehyde 3-phosphate dehydrogenase, can induce cartilage damage similar to osteoarthritis. MIA makes the loss of proteoglycan in cartilage and induces chondrocyte death. MIA is used as a substance for inducing osteoarthritis in this experiment.

The rotarod analysis is an assay for assessing the balance and motor coordination of the mice. One week before the test, the mice are trained on the rotating rod to keep on the rotating rod for over 3 minutes. After treatment, the motor coordination of the mice is assessed through the time for which the mice keep on the rotating rod.

PRP is a concentrate of platelet-rich plasma protein derived from whole blood centrifuged to remove red blood cells. PRP includes various growth factors so that it is considered to contribute to the recovery of cartilage, tendon, ligament, and so on. In this experiment, PRP is derived from Sprague-Dawley (SD) rats. Firstly, the rats are anesthetized with 3% pentobarbital sodium at a dosage of 30 mg/kg. Next, about 7 to 8 mL of whole blood is drawn from the rats using a syringe including anticoagulant, acid citrate dextrose, wherein the concentration of the platelets in the whole blood is 0.5×10⁹ to 1.5×10⁹ of platelets per milliliter. The blood is transferred to a 15 mL centrifuge tube and centrifuged at 150 g for 10 min at room temperature for the first centrifugation, and the platelets and plasma are collected to another 15 mL centrifuge tube and centrifuged at 1500 g for 10 min at room temperature for the second centrifugation. After the second centrifugation, three quarters of the supernatant at the upper part is removed, and the remained one quarter at the bottom part is the PRP used in this experiment. PRP as described here includes 1×10⁹ platelets per milliliter and various growth factors, and the type and the amount of the growth factors are as follows: TGF-b1 (≥ 7767 pg/mL), PDGF-AA (≥ 9.73 pg/mL), PDGF-AB (≥ 10 pg/mL), and PDGF-BB (≥ 590 pg/mL).

The following describes the experimental procedure. The mice are separately housed in cages for several days for adaptation in order to prevent the mice from being nervous and anxious due to the environment to affect the experimental result. The rotarod test is performed on the mice before injecting MIA as the data of day 0 and as the comparative base for subsequent tests. The mice are administrated with 0.25 mL 4% chloral hydrate 10 minutes before surgery and administrated with isoflurane during surgery to keep anesthetized and prevent awaking. 0.1 mg MIA is dissolved in 10 µL normal saline and injected into the articular cavity of the mice by a 30 G (gauge) needle. On day 7 after injecting MIA, the composition of the embodiments of the present disclosure is injected into the articular cavity of the mice by the same injection means. On days 7 and 14, the rotarod test is performed, the test time is at most 1200 sec, and the rotation speed is 20 rpm.

The experiment results of the motor coordination of the mice are shown in Table 4. The comparative group is the group in which the mice are induced to suffer from osteoarthritis and are not treated with the composition of the embodiments of the present disclosure. PRP group is the group in which the mice are induced to suffer from osteoarthritis and are treated with PRP. The test group is the group in which the mice are induced to suffer from osteoarthritis and are treated with the composition of the embodiments of the present disclosure including different concentrations of the mitochondria and/or 10 µL of PRP. The experiment results show that compared to day 0, which is not affected by osteoarthritis, the motor coordination of the mice in each group decreases to 30 to 40%. On day 14, the comparative group recovers to only 40%, and PRP group and the test group recover to 50% or more. In addition, compared to PRP group (10 µL of PRP) and Embodiment 4 (15 µg of the mitochondria), the motor coordination of the mice in Embodiment 6 (15 µg of the mitochondria and 10 µL of PRP) significantly increases. The recovery of the motor coordination of the mice indicates pain, inflamed swelling, or rigidity caused by cartilage damage or osteoarthritis are relieved. From the above experiment results, it can be seen that the composition of the embodiments including the mitochondria may improve the motor coordination of the animals suffering from osteoarthritis, and the effect increases with the concentration of the mitochondria increased. In addition, the composition including the mitochondria and PRP has a more excellent effect than the composition including only mitochondria or only PRP.

Some literature recites that the area of cartilage tissue in the articular cavity of mouse is about 1896.91 µm². This experiment confirms that pain, inflamed swelling, or rigidity caused by cartilage damage or osteoarthritis are effectively relieved by administrating 15 µg to 40 µg of mitochondria to the articular cavity of the mice. Accordingly, after conversion, the effective dose of the mitochondria may be 7.9 mg to 2.11 g per 1 cm² of cartilage tissue, and preferably 7.9 mg to 21.1 mg per 1 cm² of cartilage tissue.

**Table 4**

| Group | | Mitochondria (µg) | PRP (µL) | Day 0 | Day 7 | Day 14 |
|---|---|---|---|---|---|---|
| Control group | | - | - | 100% | 36% | 40% |
| PRP group | | - | 10 | 100% | 33% | 58% |
| Test group | Embodiment 4 | 15 | - | 100% | 30% | 55% |
| | Embodiment 5 | 40 | - | 100% | 32% | 80% |
| | Embodiment 6 | 15 | 10 | 100% | 32% | 84% |

In view of the above description, by providing mitochondria to cartilage, the mitochondrial function of the chondrocytes can be improved so as to increase the repair ability of the chondrocytes and repair cartilage damage caused by aging of the chondrocytes, and further relieve pain, inflamed swelling, or rigidity caused by cartilage damage or osteoarthritis, thereby achieving the purpose of treating osteoarthritis.

## Claims

1. A use of mitochondria in manufacturing a composition for repairing cartilage damage.

2. A use of mitochondria in manufacturing a composition for relieving osteoarthritis.

3. The use of claim 1, wherein repairing cartilage damage is to repair the cartilage damage caused by aging of chondrocytes.

4. The use of claim 1, wherein repairing cartilage damage is to improve the mitochondrial function of chondrocytes so as to increase the repair ability of the chondrocytes.

5. The use of claim 1, wherein the composition relieves pain or inflamed swelling caused by the cartilage damage.

6. The use of claim 2, wherein relieving osteoarthritis is to repair cartilage damage caused by aging of chondrocytes in joints.

7. The use of claim 2, wherein relieving osteoarthritis is to improve the mitochondrial function of chondrocytes in joints so as to increase the repair ability of the chondrocytes in the joints.

8. The use of claim 2, wherein the composition relieves pain or inflamed swelling caused by the osteoarthritis.

9. The use of claim 1 or 2, wherein the concentration of the mitochondria in the composition is 1 µg/mL to 4000 µg/mL.

10. The use of claim 1 or 2, wherein the composition further comprises platelet-rich plasma.

11. A composition, comprising mitochondria and growth factors from blood.

12. The composition of claim 11, wherein the growth factors is from platelet-rich plasma.

13. The composition of claim 11, wherein there are 10 µg to 40 µg of the mitochondria per 10 µL of the platelet-rich plasma in the composition.
